# EUROPEAN PATENT SPECIFICATION

(11) **EP 0 878 179 B1**
(45) Date of publication and mention of the grant of the patent: **02.04.2003**
(21) Application number: 98303837.3
(22) Date of filing: 15.05.1998
(51) Int. Cl.: A61F 13/06

(54) **Multi-layer compression bandage**
Mehrschichtige Kompressionsbandage
Bandage compressif multicouche

(30) Priority: 16.05.1997 US 46909 P
(43) Date of publication of application: 18.11.1998
(73) Proprietor: ETHICON, INC., Somerville, NJ 08876 (US)
(72) Inventor: Suehr, Susan, Belle Mead, NJ 08502 (US); Boersma, Terry, Arlington, TX 76006 (US); Sawicki, Rich, Plano, TX 75075 (US)
(74) Representative: Fisher, Adrian John

(56) References cited:
- WO-A-94/08541
- WO-A-94/12133
- FR-A- 2 542 191
- US-A- 3 299 890
- US-A- 3 678 933
- US-A- 3 871 378
- US-A- 4 146 027
- US-A- 4 349 020

## Description

### Field of the Invention

The present invention pertains to compression bandages, and in particular to multi-layer compression bandages.

### Background of the Invention

Multi-layer compression bandages provide the most current and successful method for handling venous leg ulcers. However, improper compression bandaging has been know to produce leg ulcers. In a paper entitled "Padding Bandage Performance Analysis" presented at the fifth European Conference on Advances In Wound Management, November 21-24, 1995, Rigby et al. describe how improper compression bandaging, caused by substrates with low compressibility, can produce ulcers at the tibia region of the leg due to the high pressure exerted over the small radius of the tibia. They opine that the lack of subcutaneous tissue in the region exasperates the problem by leaving that area of the leg without any natural padding of its own. They further demonstrate that polyester components are better suited for this than absorbent components such as rayon because of the ability of polyester to compress greatly.

To alleviate such problems, compression bandage systems typically employ multiple layers including a padding layer between the skin and the compression layer or layers. A four layer bandage compression therapy was developed in the late 1980's by Charring Cross Hospital in the United Kingdom as an elastic replacement for the Unna boot used theretofor. (The Unna boot is a nonstretchable zinc oxide paste impregnated gauze.) This four layer system included an inner layer of orthopedic wool to absorb exudate and alleviate pressure points, a crepe bandage to increase absorbency and smooth out the orthopedic wool layer so as to avoid pressure points, a highly elastic conformable compression bandage to provide graduated compression, and a cohesive elastic bandage to provide additional compression and retain the system in place for upwards of one week. The four layer system is not optimal because the first layer is difficult to apply, it has little integrity because processing of absorbent fibers compresses them significantly, and specific training must be given to avoid causing pressure points. If not applied properly, the system slips significantly and sustained compression is not maintained. Over time the four layer system slips more and is uncomfortable to the patients and their compliance to keep the bandage on becomes difficult.

The study by Rigby et al. shows the importance of proper padding under the compression bandage. Their preferred padding layer comprised an all polyester needle punched and thermo-bonded nonwoven fabric. It was shown to reduce pressure on the tibia during compression bandaging.

Recently, practitioners have also used DELTA ROLL S™ available from Johnson & Johnson Professional Products, in the United Kingdom, as the first layer in a compression bandage system. It comprises polyester fibers needle punched at about 800 to 1,000 per inch (per 25.4 mm) to a polyurethane foam substrate. The fiber side is placed in contact with the leg. While providing good padding, it provides absolutely no absorption for an oozing leg ulcer. It has been discovered that non-absorbent inner layers are unacceptable in compression bandaging. Venous ulcers typically exude large amounts of fluid-and this generally increases greatly upon application of the compression bandage. If a wound dressing sufficient to absorb the exudate were applied under a non-absorbent first layer it would expand to produce a large prominence under the compression bandage. A prominence under the pressure of the compression bandage can create pressure ulcers and are to be avoided. Without absorption, the wound exudate would lie against the skin potentially causing damage thereto and reducing the amount of time the bandage system can be worn. For instance, daily dressing changes may be required.

The present invention, in a preferred embodiment thereof, overcomes these limitations and others by providing an inner layer comprising cotton fibers lightly needle punched to a polyurethane foam substrate to producing an inner compression bandage layer having a high absorptive capacity, a high ability to compress, a high coefficient of friction against wet skin, and a high coefficient of friction against itself. The present invention overcomes the obstacles of a commercial four layer system that has an absorbent fiber bat as the first layer and a lightly stretchable crepe layer as the second component.

Additionally, the invention can optionally provide a compression bandage and a cohesive compression bandage in a resealable container which optionally also includes a non-adherent wound dressing. This preferred "kit" allows a health care provider to tailor the kit for an individual patient and conveniently transport that specifically tailored compression bandage kit to the patients residence in a home health care situation.

FR-A-2542191 makes known a bandaging material comprising an upper layer of a compressed polyurethane foam which presents an upper face of the bandaging material, a nonwoven, inner fabric layer bonded to a lower face of the upper foam layer, and a lower layer which presents a lower face of the bandaging material. The lower layer is formed from an adhesive material which extends from the lower face through the inner fabric layer and penetrates into the lower face of the upper foam layer thereby bonding the inner and upper layers together.

US-A-4146027 makes known a wound dressing having a top layer of a foam which is self-bonded to a nonwoven absorbent layer. In use, the foam surface is the skin-contacting layer of the wound dressing.

### Summary of the Invention

A compression bandage system according to the present invention has the features set forth in claim 1 appended hereto.

Preferably, the foam comprises a polyurethane, more preferably an ester based polyurethane, and most preferably is formed of a polyester polyol and toluene diisocyanate.

Preferably, the nonwoven web comprises carded fibers, especially cotton fibers. The cotton fibers are present, preferably, in an amount at least 70% cotton by weight and more preferably are essentially all of cotton. The carded fibers may optionally comprise upwards of 30% polyethylene terephthalate fibers.

Preferably, the fibers are needle punched to the polyurethane foam at less than 500 needles per inch (per 25.4 mm), more preferably at less than 200 needles per inch (per 25.4 mm), and most preferably at less than 100 needles per inch (per 25.4 mm).

Preferably, the fibers comprise cotton and are present at between 3.5 and 4.5 ounces per square yard (118.68 and 152.57 g/m²).

Preferably, the coefficient of friction between a surface of the foam and a surface of the fibers is at least 1.5 gm/gm, more preferably is at least 1.8 gm/gm, and most preferably is at least 2.3 gm/gm.

Preferably, the inner layer exhibits a stretch capability of at least 50% and more preferably of at least 75%. Preferably, the inner layer exhibits a compression in use of at least 10% after one hour of 40mm Hg (5.33 kPa) compression and at least 20% after eighteen hours of 40mm Hg (5.33 kPa) compression.

The outer layer may comprise one elastic layer, or more than one elastic layer.

The compression bandage system according to the present invention may further comprise a wound dressing for covering a venous ulcer, with the inner layer for application over the wound dressing and against a patient's skin and the outer layer for application over the inner layer. A resealable enclosure contains the wound dressing, the inner layer and the outer layer and has a sealing means thereon for opening and closing the enclosure. Thus, a user may add components to the system, take components from the system or exchange like components in the system and easily transport the complete system to a point of use, such as to a home health care site. Preferably, the enclosure comprises a plastic bag and the sealing means comprises an interlocking tongue and groove seal.

### Brief Description of the Drawings

FIG. 1 is a photo-micrograph of an inner layer of a compression bandage system according to the present invention;
FIG. 1A is a photo-micrograph of a foam substrate forming a portion of the inner layer of FIG. 1;
FIG. 2 is a top plan view of a segment of elastic bandage;
FIG. 3 is a top plan view of a segment of the bandage of FIG. 2 in a stretched condition;
FIG. 4 is a top plan view of the segment of FIG. 2 in. an overstretched condition;
FIG. 5 is a fragmentary pictorial view of a segment of the knitted compression bandage illustrating the placement of an indicator yarn therein;
FIG. 6 is an elevational view of a compression bandage system according to the present invention;
FIG. 7 is an elevation view of the layer of FIG. 1 being applied to a patient's leg, specifically the foot area;
FIG. 8 is an elevation view of the application process as shown in FIG. 7, with the first layer completely applied;
FIG. 9 is an elevation view of the application of the second layer of the compression bandage system according to the present invention;
FIG. 10 is an elevation view of the completed application of the second layer as of the compression bandage system; and
FIG. 11 is an elevation of the third layer of the compression bandage system according to the present invention overwrapping the first and second layers thereof.

### Detailed Description

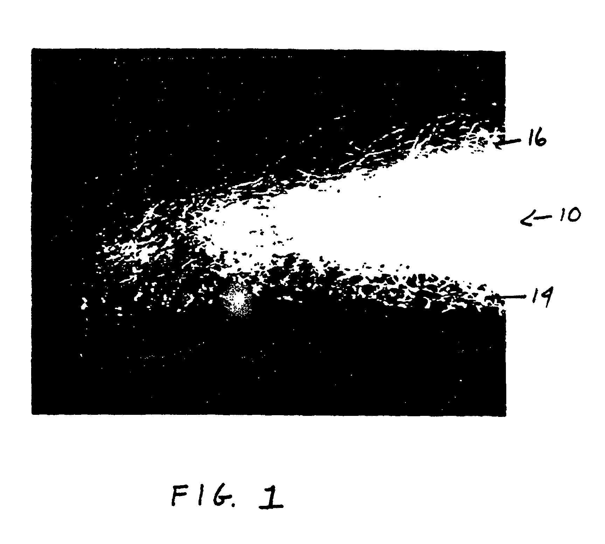

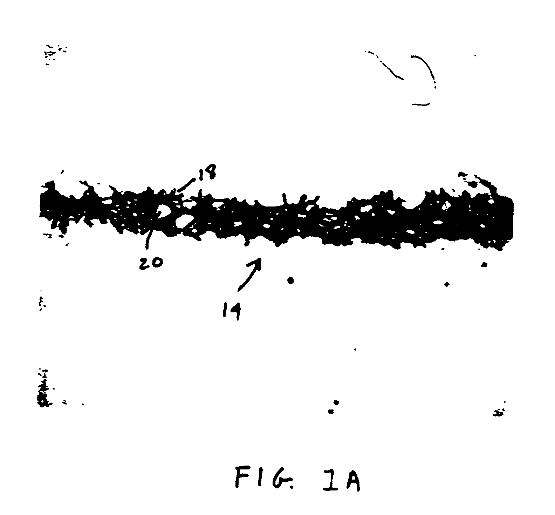

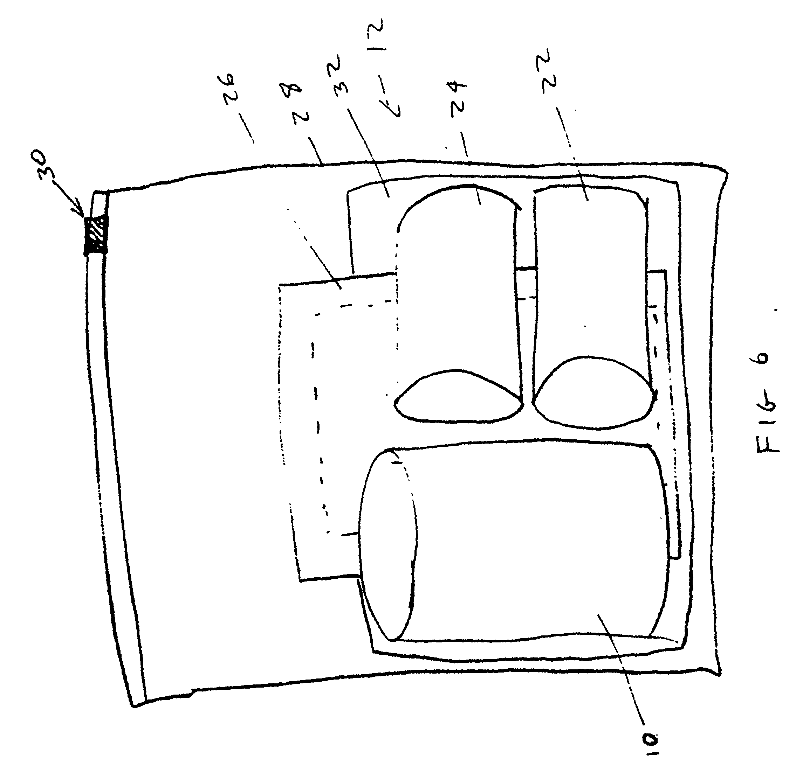

FIG. 1 illustrates an inner layer 10 of a compression bandage system 12(see FIG. 6). The inner layer 10 comprises a polyurethane foam substrate 14 and a web of carted absorbent fibers 16. The foam substrate 14 preferably has a thickness of between 15 and 60 mils (381 and 1524 µm), and more preferably between 20 and 50 mils (508 and 1270 µm), and most preferably of 30 to 40 mils (762 to 1016 µm).

A polyurethane which is cut to the thickness dimension is preferred over foams formed to the thickness dimension due to the jagged edges 18 formed by the slicing open of individual cells 20 forming the polyurethane substrate 14(see FIG. 1A). Ester based polyurethanes are preferred for their generally stiffer nature and smaller cell size which provide more jagged edges 18 of sufficient rigidity to produce a high coefficient of friction against the absorbent fiber web 16. A particular well suited polyurethane is available from William T. Burnett & Company Inc., of Jessup, Maryland in the form of their product number S82FF90. It has a minimum density of 1.3 lbs. per cubic foot (24.03 kg/m³), a minimum tensile strength of 22 lbs. per square inch (15.47 x 10³ kg/m²) and an average tensile strength of 28 lbs. per square inch (19.69 x 10³ kg/m²), a minimum ultimate elongation of 300%, and an average ultimate elongation of 400%. A pressure of 0.5 lbs. per square inch (3.48 kPa) produces a 50% deflection. All tests are performed in accordance with ASTM-3574.

The absorbent fiber web 16 preferably has a basis weight of 3.0 to 5.0 ounces per square yard (101.72 to 169.53 g/m²), and more preferably a density of 3.5 to 4.5 ounces per square yard (118.68 to 152.57 g/m²). Preferably the fiber comprises cotton. It has been found that cotton exhibits a high coefficient of friction in contact with wet skin. Rayon, although chemically similar to cotton, typically does not exhibit such a high coefficient of friction in contact with wet skin. However, some rayons are produced with a physical structure to more closely imitate cotton fibers, such as a tri-lobal shaped rayon or TENCEL™ which is available from Courtaulds in Mobile, Alabama. Mixing cotton fibers in with the rayon fibers can increase the coefficient of friction with wet skin. Of course other absorbent fibers exhibiting acceptable coefficients of friction with wet skin may be substituted by those of skill in the art. Although repeatable measurements of the coefficient of friction against wet skin are difficult, the absorbent fiber web 16 should exhibit a coefficient of friction against itself of at least 0.7 gm/gm and more preferably of at least 0.9 gm/gm.

To provide directional memory, a small proportion of PET fibers may be added. Preferably these fibers comprise less than 30%, and more preferably less than 15 %

The absorbent fiber web 16 is lightly needle punched to the polyurethane substrate 14. Preferably, the needle punching is performed at less than 500 needles per inch (25.4 mm), more preferably at less than 200 needles per inch (25.4 mm) and most preferably at less than 100 needles per inch (25.4 mm). A higher density of needles in the needle punch process tends to compress the absorbent fiber web 16 and reduce its effectiveness as a cushioning layer.

Overall, the inner layer should stretch by at least 50% in length, preferably by 75% in length and most preferably by 100% in length. Also, the coefficient of friction between the polyurethane foam substrate 14 and the absorbent fiber web 16 in the completed inner layer 10 is important. As will be more fully described hereinafter, the inner layer 10 is wrapped about a limb with the absorbent fiber web 16 facing the skin in overlapping wraps so that after the layer has been wrapped once around the limb it overlaps by 50% the polyurethane substrate 14 which is facing outwardly from the limb. Accordingly, the polyurethane substrate 14 and absorbent fiber web 16 are in contact with each other in successive wraps of the bandage. A high coefficient of friction between these two surfaces ensures that the bandage will not slip upon itself during use. Accordingly, the coefficient of friction between the polyurethane substrate 14 and absorbent fiber web 16 should be at least 1.5 gm/gm, and more preferably 1.8 gm/gm, and most preferably at least 2.3 gm/gm.

To provide good padding, the inner layer should compress to a certain degree under the degree of compression it will experience in use, without actually flattening. It is most important that it maintain this compression profile over time so that it does not flatten during extended use, which typically lasts 7 to 9 days or perhaps longer. Under a sustained compression of 40 mm Hg (5.33 kPa), the inner layer 10 should compress by at least 10% and at not more 30%. It should stay within this range over a period of sustained compression. Preferably, it should exhibit at least 10% compression one hour after forces applied and at least 20% 18 hours after force is applied.

A sample of the inner layer 10 was prepared according to the following specifications: 4.0 oz/SQYD (135.62 g/m²) cotton, 23 mils (584.2 µm) thick polyurethane foam (ester based), needle punched at 200 needles/ inch (per 25.4 mm), as herein described.

It was compared against a sample of DELTAROLL S™, which has the following specifications: 1.4 oz/SQYD (47.47 g/m²) polyester, 22 mils thick polyurethane foam(ester based), needle punched at 1000 needles/ inch (per 25.4 mm).

The results of the evaluation are reported in Table 1 below. Friction was determined using ASTM test method D1894 on an INSTRON Model Number 1122 Tester using a 200 gm weight sled. Results are recorded in grams of force per grams of weight.

**TABLE 1**

| | Inner layer 10 @ 100 needles/inch (25.4 mm) | Inner layer 10 @ 200 needles/inch (25.4 mm) | Commercial four layer system | Delta Roll "S"™ |
|---|---|---|---|---|
| % stretch | 52% | 73% | 33% | 50% |
| Fiber to foam coefficient of friction | 2.3 gm/gm | 1.5 gm/gm | 0.8 gm/gm | 2.2 gm/gm |
| Fiber to fiber coefficient of friction | 0.9 gm/gm | 0.7 gm/gm | 0.7 gm/gm | 0.7 gm/gm |
| One hour compression | 13% | 10% | 6% | 11% |
| 18 hour compression | 18% | 23% | 19% | 22% |

The inner layer 10 was also compared with a commercially available four layer compression bandage system of similar construction to the above described four layer system in connection with Charring Cross Hospital. By having a high coefficient of friction against wet skin and itself, the inner layer 10 was demonstrated to slip less over 7 days than the commercially available four layer system. In a test of 35 adults wearing both bandages where each bandage was alternated between left and right legs, slipped significantly less at the 95% confidence level than an available commercial four layer system. This invention's slippage on the average was 2.5 inches (63.5 mm) after 7 days and the available four layer system slipped 2.7 inches (68.58 mm) after 7 days of wear. Statistics at the 95% confidence level predict that this invention's expected slippage is between 2.36 and 2.6 inches (58.42 and 66.04 mm), whereas the available four layer system's expected slippage is between 2.6 and 2.86 inches (66.04 and 72.65 mm). Slip was measured from the original location of the top of the bandage at the knee to the position below that to which the top of the bandage slipped.

More significantly, the slip characteristics of the present invention left little or no gaps in the inner layer after a week of wear. When it slipped, it did so uniformly over the length of the leg, the successive wraps increasing their overlap slightly from the 50% overlap as applied. In contrast, the four layer system slipped in a less even fashion, frequently leaving gaps at the ankle as the bandage inner layer slipped from the ankle to below the foot, and thereby leaving bony prominences unprotected by the inner layer.

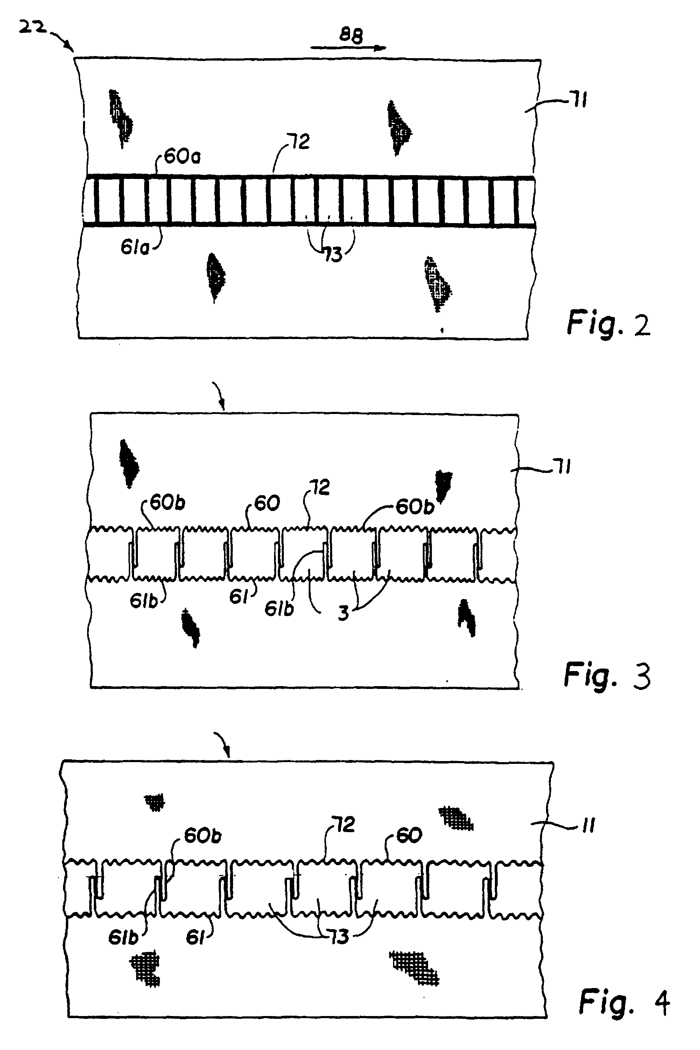

FIG. 2 illustrates a segment 40 of knitted elastic bandage 71 forming a second layer 22 of the compression bandage system 12. The elastic bandage 71 may be any of various commercially available knit or woven materials formed on various length, width, densities, etc., for use as compression bandages or windings. A typical knit bandage and method of making same is disclosed in United States Patent No. 4,665,909.

As shown in FIG. 2 indicator yarns 60a and 61a are positioned within the knit fabric 71 to define a continuous pattern 72 comprised of adjoining rectangles 73 extending the length of the strip. The yarn which forms the continuous pattern 72 is contained within the knit fabric in two parallel rows, each of which parallels the wales of the strip. At predetermined spaced intervals, each of the parallel yarns 60a, 61a is deviated toward the other (as more clearly illustrated in FIGS. 3 and 4) so that the opposed excursions merge to define parallel interconnection bars 60b, 61b between the parallel yarn base lines. The parallel bars, in cooperation with the-parallel yarn base lines, define a series of rectangles 73.

The knitted strip is formed in the stretched condition. Thus, the indicator yarn is positioned parallel with the wales of the knit in its fully extended condition. However, as the elastic bandage contracts, the indicator yarn is compressed axially to form rather enlarged indicator pattern lines 60a, 61a as shown in FIG. 2. As shown in FIG. 4, the indicator yarn 60, 61 in each row deviates inwardly toward the other row may overlap as shown. When the bandage is stretched, the overlap of the yarn forming an indicator bar may become visually apparent.

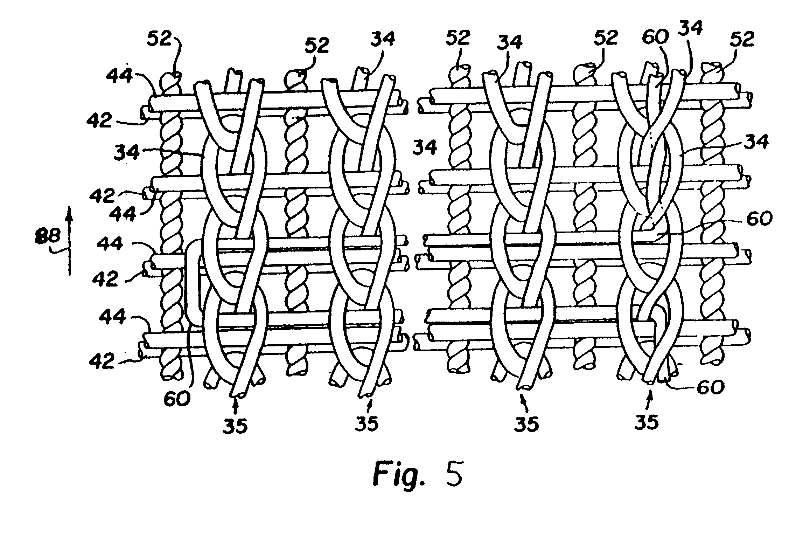

Inclusion of an indicator yarn in a knitted strip is shown in detail in FIG. 5 which illustrates formation of a knit yarn as disclosed in US patent No. 4,665,909, *supra*. As shown in FIG. 5, the fabric 71 is formed with a plurality of knitted warp yarns 34 forming individual, unconnected wales or columns 35 extending the full length of the fabric strip parallel with each other in direction 88. Front and back strands of weft or filling yarn 44 and 42, respectively, are interwoven or floated within the knitted wales of the warp yarns in transverse direction between each side edge of the fabric to define a plurality of aligned front and back courses. Each pick or course of front and back weft yarn passes through and engages each loop of warp yarn aligned with that course of fabric. A plurality of secondary warp strands 52 are longitudinally interlayed or folded between the front and back courses of the weft yarn and between and parallel with each of the wales of the primary warp yarn. The front courses of the weft yarn are thus spaced from the back courses of the weft yarn by the thickness of the secondary warp strands 52. The secondary warp yarn 52 is elastic and may be rubber or synthetic material. The secondary warp stands enable the fabric to be stretched and to exert compressive force when wrapped around a body part or the like.

Parallel strands of colored yarn 60, 61 (only yarn 60 is shown in FIG. 5) are laid within the front face of the knit fabric parallel with one column. Preferably, each indicator yarn 60, 61 is included in one column of knitted warp as shown in FIG. 5.

As illustrated in FIG. 5, a colored yarn 60 is included in the column 35 of warp 34 and thus extends parallel with longitudinal direction 88. However, at preselected spaced intervals the indicator yarn 60 deviates laterally across the wales 35 a predetermined excursion distance (parallel with weft yarn 44) and then, within a single stitch, returns to the original column. Each excursion forms a parallel deviation or interconnection bar 60b, 61b as shown in the stretched segments of FIG. 2 and FIG. 3. In the preferred embodiment, the excursions 60b, 61b are formed in adjacent stitches so that they may overlap and thus form a continuous deviation or indicator bar. As noted above, the indicator yarn is inserted while the knot fabric is being formed in the stretched condition. Thus, when the knitted fabric contracts, the adjacent deviation bars 60b, 61b collapse longitudinally to form a pattern visually perceived as a single enlarged bar.

A single strand of yarn is used as the indicator yarn in each base line 60a, 60b. However, multiple strands may be used if desired. The indicator yarn 60, 61 is merely used to form a visual marker. Thus, the term "colored" as used herein merely indicates that the visually perceived color of the indicator yarn 60, 61 is sufficiently contrasted with the visually perceived color of the knit fabric yarn so that the user may identify the pattern formed thereby. A four inch (101.6 mm) wide 50% compression bandage was formed which produces 25 mm Hg (3.33 kPa) compression at the ankle portion of a human leg with the following:
2 strands nylon (70/2)
28 strands polyester (1/150 d.) (740.74 µtex)
27 strands polypropylene (210 d.) (23.33 tex)
4 strands cotton (30/1)
56 strands rubber (60 ga.)
An additional two (2) strands of blue polyester (150 d.) (16.66 tex) were used to form the indicator. The parallel indicator strands 60, 61 were inserted 5/8 inch (15.875 mm) apart to form parallel tracks on either side of the centerline with interconnection bars 60b, 61b spaced 5/8 inch apart (15.875 mm) (inside dimensions) at 50% compression. Since this fabric was knitted at 210% stretch, the deviations were spaced a proportional distance apart during fabrication, thus forming rectangles elongated in the longitudinal direction when formed. However, in the relaxed condition the indicator yarns are compressed and the square become rectangles elongated in the transverse direction. Nevertheless, when the bandage strip is stretched to 50% compression, each rectangular design becomes a 5/8 include by 5/8 inch (15.875 mm) square.

Of course, any suitable elastic compression bandage can be substituted therefore. However, the bandage should provide the required degree of compress, breathability and patient comfort. Lightweight, long stretch, class 3C high compression, cotton-polyester knitted bandages are preferred. Class 3C refers to the Thomas Classification System and provides high compression of between 25 to 35 mm Hg (3.33 to 4.66 kPa).

The third layer 24 comprises a nonwoven fabric with SPANDEX elastic yarns bonded with a natural rubber cohesive latex base system. This layer provides about 40% of the compression required for the overall system 12. It is composed of about 35% nonwoven nylon fibers, 63% latex cohesive and 2% elastic fibers. It provides a long stretch and the latex cohesive allows it to stick upon itself without requiring clips or other attachment media. A suitable cohesive compression bandage is available from Andover coated products of Salisbury, Massachusetts under the tradename CO_FLEX™.

Generally, the ulcerated portion of the leg should be dressed with a non-adherent wound dressing 26 so that the inner layer 10 does not stick to the ulcer. A preferred wound dressing is the ADAPTIC™ non-adhering dressing available from Johnson & Johnson Medical, Inc., of Arlington, Texas which comprises a non-adherent cellulose acetate net impregnated with a petrolatum emulsion.

Each of these components can be conveniently packaged within a resealable container 28 such as a resealable plastic bag, preferably with a zipper like sealing mechanism 30. A zipper type tongue and groove ZIPLOCK™ seal is preferred. The inner layer 10, the second layer 22, and the third layer 24, are preferably rolled and individually wrapped. In a preferred embodiment, the inner layer 10 is provided in a 4 inch (101.6 mm) by 156 inch (3.96 m) dimension (unstretched), the second layer in a 4 inch (101.6 mm) by 90 inch (2.29 m) dimension (unstretched)and the third layer in a 4 inch (101.6 mm) by 216 inch (5.49 m) dimension (stretched). A product insert 32 with instructions for applying the wound dressing is also included within the enclosure 28.

As provided by the instructions, the wound dressing is applied as follows. First, the wound is cleaned and dressed as appropriate. Preferably, the non-adherent dressing 26 is applied thereto. Other dressing types may be substituted therefore, such as hydropolymer dressings, collagen-alginate wound dressings, foam dressings, hydrocoloid dressings, or collagen wound gels.

Turning now to FIG. 7, after the wound dressing 26 is applied, the inner layer 10 is spirally wound onto the affected limb, in this case a leg. The winding starts from the base of the toes using a spiral technique at 50% overlap and continues to just below the knee. Only slight tension need be applied, merely enough to make the inner layer adhere to itself. As described before, the absorbent fiber web 16 is placed in contact with the skin and overlaps in each spiral wrap onto the polyurethane substrate 14. The jagged edges 18 of the cut polyurethane (see FIG. 1A) adhere against the fiber web 16 to prevent slippage of the inner layer. FIG. 8 shows the complete wrapping of the inner layer 10. Any excess should be removed and a free end attached with an appropriate medical grade tape.

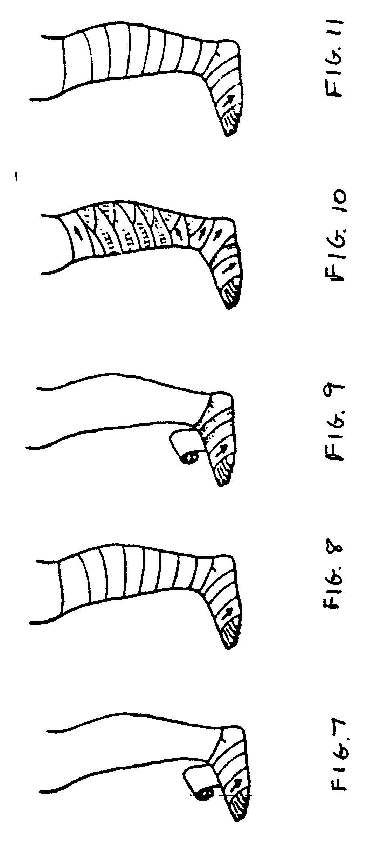

Over the inner layer, the second elastic compression layer 22 is applied. A spiral technique is used between the base of the toes and the ankle as illustrated in FIG. 9, and optionally above the ankle a figure 8 technique as illustrated in FIG. 10 may be applied for enhanced compression. If less compression is desired the spiral technique may be applied all the way to the knee. A 50% stretch with a 50% overlap Is preferred. Over the second layer, the third compression cohesive layer 24 is applied in a spiral technique with a 50% extension and 50% overlap. Any excess bandage should be removed to avoid any overlap at the knee. The self adhering nature of the outer cohesive bandage allows it to stay in place without further restraints. The compression bandage system 12 delivers sustained compression over a period of 7 to 9 days.

While the invention has been described with regard to a particular embodiment thereof, those skilled in the art will understand, of course, that the invention is not limited thereto since modifications can be made by those skilled in the art, particularly in light of the foregoing teachings. Reasonable variation and modification are possible within the foregoing disclosure of the invention without departing from the scope of the invention as defined by the appended claims.

## Claims

1. A compression bandage system (12) comprising:-
(a) an inner skin-facing layer (10) comprising:-
(i) a first ply (14) of foam,
(ii) a second ply (16) of an absorbent nonwoven web,
(iii) a first outermost surface, and
(iv) a skin-facing second outermost surface spaced in opposed relation from the first outermost surface and formed by the nonwoven absorbent web of the second ply; and
(b) an elastic outer layer (22) which, in use, overlies the first outermost surface of the inner layer,
wherein the inner and outer layers are elongated so as to be capable of being wound about a patient's limb.

2. A compression bandage system according to claim 1 wherein the foam comprises a polyurethane.

3. A compression bandage system according to claim 2 wherein the foam comprises an ester based polyurethane.

4. A compression bandage system according to claim 3 wherein the polyurethane foam is formed of a polyester polyol and toluene diisocyanate.

5. A compression bandage system according to claim 1 wherein the nonwoven web comprises carded fibers.

6. A compression bandage system according to claim 5 wherein the carded fibers comprise cotton.

7. A compression bandage system according to claim 5 wherein the carded fibers comprise at least 70% cotton by weight.

8. A compression bandage system according to claim 5 wherein the carded fibers consist essentially of cotton.

9. A compression bandage system according to claim 5 wherein the carded fibers comprise polyethylene terephthalate fibers in an amount no more than 30% by weight.

10. A compression bandage system according to claim 5 the fibers are needle punched to the polyurethane foam.

11. A compression bandage system according to claim 10 wherein the fibers are needle punched at less than 500 needles per inch (25.4 mm).

12. A compression bandage system according to claim 11 wherein the fibers are needle punched at less than 200 needles per inch (25.4 mm).

13. A compression bandage system according to claim 12 wherein the fibers are needle punched at less than 100 needles per inch (25.4 mm).

14. A compression bandage system according to claim 12 wherein the fibers comprise cotton and are present at between 3.5 and 4.5 ounces per square yard (118.68 and 152.57 g/m²).

15. A compression bandage system according to claim 10 wherein the coefficient of friction between a surface of the foam and a surface of the fibers is at least 1.5 gm/gm as measured using ASTM test method D1894.

16. A compression bandage system according to claim 15 wherein the coefficient of friction between the surface of the foam and the surface of the fibers is at least 1.8 gm/gm as measured using ASTM test method D1894.

17. A compression bandage system according to claim 15 wherein the coefficient of friction between the surface of the foam and the surface of the fibers is at least 2.3 gm/gm as measured using ASTM test method D1894.

18. A compression bandage system according to claim 1 wherein the inner layer has the capability of stretching at least 50% without destruction thereof.

19. A compression bandage system according to claim 1 wherein the inner layer has the capability of stretching at least 75% without destruction thereof.

20. A compression bandage system according to claim 1 wherein the inner layer exhibits compression of between 10% and 30% after one hour of compression of 40mm Hg (5.33 kPa) from the outer layer.

21. A compression bandage system according to claim 1 wherein the inner layer exhibits compression of between 20% and 25% after eighteen hours of compression of 40mm Hg (5.33kPa) from the outer layer.

22. A compression bandage system according to claim 1 wherein the elastic outer layer comprises more than one independent elastic layer (22, 24).

23. A compression bandage system according to any one of the preceding claims further comprising:
a wound dressing for covering a venous ulcer; and
a resealable enclosure (28) containing the wound dressing, the inner layer and the outer layer and having a sealing means (30) thereon for opening and closing said enclosure whereby a user may add components to the system, take components from the system or exchange like components in the system and easily transport the complete system to a point of use.

24. A compression bandage system according to claim 23 wherein the enclosure comprises a plastic bag.

25. A compression bandage system according to claim 24 wherein the sealing means comprises an interlocking tongue and groove seal.

## Patentansprüche

1. Kompressionsbandagesystem (12) umfasssend
(a) eine der Haut zugewandte Innenlage (10) ihrerseits umfassend:
(i) eine erste Schicht (14) aus Schaum,
(ii) eine zweite Schicht (16) aus einem absorbierenden Vlies,
(iii) eine erste äußerste Oberfläche und
(iv) eine der Haut zugewandte zweite äußerste Oberfläche im Abstand von der ersten äußersten Oberfläche, dieser gegenüberliegend, und gebildet aus dem absorbierenden Vlies der zweiten Schicht und
(b) eine elastische Außenlage (22), welche beim Gebrauch die erste äußerste Oberfläche der Innenlage bedeckt, wobei die Innen- und die Außenlage langgestreckt sind, so daß sie um ein Glied eines Patienten gewickelt werden können.

2. Kompressionsbandagesystem nach Anspruch 1, bei welchem der Schaum aus Polyurethan besteht.

3. Kompressionsbandagesystem nach Anspruch 2, bei welchem der Schaum aus Polyurethan auf Ester-Basis besteht.

4. Kompressionsbandagesystem nach Anspruch 3, bei welchem der Polyurethan-Schaum aus Polyester-Polyol und Toluen-Diisocyanat besteht.

5. Kompressionsbandagesystem nach Anspruch 1, bei welchem das Vlies aus kardierten Fasern besteht.

6. Kompressionsbandagesystem nach Anspruch 5, bei welchem die kardierten Fasern aus Baumwolle bestehen.

7. Kompressionsbandagesystem nach Anspruch 5, bei welchem die kardierten Fasern zu mindestens 70 Masse-% aus Baumwolle bestehen.

8. Kompressionsbandagesystem nach Anspruch 5, bei welchem die kardierten Fasern im wesentlichen aus Baumwolle bestehen.

9. Kompressionsbandagesystem nach Anspruch 5, bei welchem die kardierten Fasern nicht mehr als 30 Masse-% Polyethylen-Terephtalat-Fasern enthalten.

10. Kompressionsbandagesystem nach Anspruch 5, bei welchem die Fasern mit dem Polyurethan-Schaum nadelverfilzt sind.

11. Kompressionsbandagesystem nach Anspruch 10, bei welchem die Fasern mit dem Polyurethan-Schaum mit weniger als 500 Nadeln pro Zoll (25,4 mm) nadelverfilzt sind.

12. Kompressionsbandagesystem nach Anspruch 11, bei welchem die Fasern mit dem Polyurethan-Schaum mit weniger als 200 Nadeln pro Zoll (25,4 mm) nadelverfilzt sind.

13. Kompressionsbandagesystem nach Anspruch 12, bei welchem die Fasern mit dem Polyurethan-Schaum mit weniger als 100 Nadeln pro Zoll (25,4 mm) nadelverfilzt sind.

14. Kompressionsbandagesystem nach Anspruch 12, bei welchem die Fasern aus Baumwolle bestehen und in einer Dichte von 3,5 Unzen bis 4,5 Unzen pro Quadrat-Yard (118,68 g/m² bis 152,57 g/m²) vorliegen.

15. Kompressionsbandagesystem nach Anspruch 10, bei welchem der Reibungskoeffizient zwischen der Oberfläche des Schaumes und der Oberfläche der Fasern mindestens 1,5 g/g (gemessen nach dem ASTM-Prüfverfahren D1894) beträgt.

16. Kompressionsbandagesystem nach Anspruch 15, bei welchem der Reibungskoeffizient zwischen der Oberfläche des Schaumes und der Oberfläche der Fasern mindestens 1,8 g/g (gemessen nach dem ASTM-Prüfverfahren D1894) beträgt.

17. Kompressionsbandagesystem nach Anspruch 15, bei welchem der Reibungskoeffizient zwischen der Oberfläche des Schaumes und der Oberfläche der Fasern mindestens 2,3 g/g (gemessen nach dem ASTM-Prüfverfahren D1894) beträgt.

18. Kompressionsbandagesystem nach Anspruch 1, bei welchem die Innenlage um mindestens 50 % ohne Beschädigung dehnbar ist.

19. Kompressionsbandagesystem nach Anspruch 1, bei welchem die Innenlage um mindestens 75 % ohne Beschädigung dehnbar ist.

20. Kompressionsbandagesystem nach Anspruch 1, bei welchem die Innenlage nach einer Stunde einer Druckausübung von 40 mm Quecksilbersäule (5,33 kPa) auf die Außenlage eine Kompression von 10 % bis 30 % aufweist.

21. Kompressionsbandagesystem nach Anspruch 1, bei welchem die Innenlage nach einer Stunde einer Druckausübung von 40 mm Quecksilbersäule (5,33 kPa) auf die Außenlage eine Kompression von 20 % bis 25 % aufweist.

22. Kompressionsbandagesystem nach Anspruch 1, bei welchem die elastische Außenlage mehr als eine elastische Lage (22, 24) aufweist.

23. Kompressionsbandagesystem nach einem der bisherigen Ansprüche, welches weiterhin umfaßt:
einen Wundverband zum Abdecken von Venengeschwüren und
eine abnehmbare Hülle (28), welche den Wundverband, die Innenlage und die Außenlage enthält und eine Abdichtungseinrichtung (30) aufweist, um die Hülle zu öffnen und zu verschließen, wodurch ein Benutzer dem System Komponenten hinzufügen, Komponenten aus diesem entnehmen oder ähnliche Komponenten austauschen sowie das komplette System leicht zur Anwendungsstelle transportieren kann.

24. Kompressionsbandagesystem nach Anspruch 23, bei welchem die Hülle ein Kunststoffbeutel ist.

25. Kompressionsbandagesystem nach Anspruch 24, bei welchem die Abdichtungseinrichtung aus einer Zunge und einem Schlitz besteht, in welchen die Zunge eingreift.

## Revendications

1. Système de bandage compressif(12) comprenant :
a) une couche intérieure (10) faisant face à la peau, comprenant :
i. une première épaisseur (14) de mousse,
ii. une deuxième épaisseur (16) d'un doublage non tissé absorbant,
iii. une première surface extérieure, et
iv. une deuxième surface extérieure faisant face à la peau, espacée, opposée à la première surface extérieure et formée par le doublage absorbant non tissé de la deuxième épaisseur ; et
b) une couche extérieure élastique (22) qui, à l'utilisation, recouvre la première surface extérieure de la couche intérieure,
dans lequel les couches intérieure et extérieure sont allongées de manière à pouvoir être enroulées autour du membre d'un patient.

2. Système de bandage compressif selon la revendication 1, dans lequel la mousse comprend un polyuréthane.

3. Système de bandage compressif selon la revendication 2, dans lequel la mousse comprend un polyuréthane fondé sur un ester.

4. Système de bandage compressif selon la revendication 3, dans lequel la mousse de polyuréthane est formé d'un polyol de polyester et d'un diisocyanate de toluène.

5. Système de bandage compressif selon la revendication 1, dans lequel le doublage non tissé comprend des fibres cardées.

6. Système de bandage compressif selon la revendication 5, dans lequel les fibres cardées comprennent du coton.

7. Système de bandage compressif selon la revendication 5, dans lequel les fibres cardées comprennent au moins 70 % en poids de coton.

8. Système de bandage compressif selon la revendication 5, dans lequel les fibres cardées sont constituées essentiellement de coton.

9. Système de bandage compressif selon la revendication 5, dans lequel les fibres cardées comprennent des fibres de téréphtalate de polyéthylène en une proportion non supérieure à 30 % en poids.

10. Système de bandage compressif selon la revendication 5, dans lequel les fibres sont insérées par piquage dans la mousse de polyuréthane.

11. Système de bandage compressif selon la revendication 10, dans lequel les fibres sont insérées par piquage selon une densité inférieure à 500 piqûres par pouce (25,4 mm).

12. Système de bandage compressif selon la revendication 11, dans lequel les fibres sont insérées par piquage selon une densité inférieure à 200 piqûres par pouce (25,4 mm).

13. Système de bandage compressif selon la revendication 12, dans lequel les fibres sont insérées par piquage selon une densité inférieure à 100 piqûres par pouce (25,4 mm).

14. Système de bandage compressif selon la revendication 12, dans lequel les fibres comprennent du coton et sont présentes en une proportion comprise entre 118,68 et 152,57 g / m² (3,5 et 4,5 onces par yard carré).

15. Système de bandage compressif selon la revendication 10, dans lequel le coefficient de friction entre une surface de la mousse et une surface des fibres est d'au moins 1,5 gm / gm, mesuré en utilisant un procédé d'essai ASTM D1894.

16. Système de bandage compressif selon la revendication 15, dans lequel le coefficient de friction entre une surface de la mousse et une surface des fibres est d'au moins 1,8 gm / gm, mesuré en utilisant un procédé d'essai ASTM D1894.

17. Système de bandage compressif selon la revendication 15, dans lequel le coefficient de friction entre une surface de la mousse et une surface des fibres est d'au moins 2,3 gm / gm, mesuré en utilisant un procédé d'essai ASTM D1894.

18. Système de bandage compressif selon la revendication 1, dans lequel la couche intérieure a une capacité d'étirement d'au moins 50 % sans destruction de celle-ci.

19. Système de bandage compressif selon la revendication 1, dans lequel la couche intérieure a une capacité d'étirement d'au moins 75 % sans destruction de celle-ci.

20. Système de bandage compressif selon la revendication 1, dans lequel la couche intérieure présente une compression comprise entre 10 et 30 % après une heure de compression à 5,33 kPa (40 mm de Hg) à partir de la couche extérieure.

21. Système de bandage compressif selon la revendication 1, dans lequel la couche intérieure présente une compression comprise entre 20 et 25 % après dix-huit heures de compression à 5,33 kPa (40 mm de Hg) à partir de la couche extérieure.

22. Système de bandage compressif selon la revendication 1, dans lequel la couche extérieure élastique est constituée par plus d'une couche élastique indépendante (22, 24).

23. Système de bandage compressif selon une quelconque des revendications précédentes, comprenant, en outre :
- un pansement pour couvrir un ulcère veineux ; et
- une enceinte susceptible d'être à nouveau rendue étanche (28), contenant le pansement, la couche intérieure et la couche extérieure et ayant des moyens d'étanchéification (30) sur celle-ci afin d'ouvrir et de fermer ladite enceinte, grâce à quoi l'utilisateur peut ajouter des composants au système, retirer des composants du système ou substituer des composants semblables dans le système et transporter aisément le système complet vers le site d'utilisation.

24. Système de bandage compressif selon la revendication 23, dans lequel l'enceinte comprend une poche en un matériau plastique.

25. Système de bandage compressif selon la revendication 24, dans lequel les moyens d'étanchéification comprennent une languette et un joint à gorge se verrouillant mutuellement.
